(19) Europäisches Patentamt
European Patent Office
Office européen des brevets

(11) **EP 2 040 803 B1**

(12) **FASCICULE DE BREVET EUROPEEN**

(45) Date de publication et mention
de la délivrance du brevet:
**11.04.2012 Bulletin 2012/15**

(51) Int Cl.:
*A61Q 19/08* $^{(2006.01)}$  *A61K 8/97* $^{(2006.01)}$
*A61K 8/99* $^{(2006.01)}$  *A61K 8/37* $^{(2006.01)}$

(21) Numéro de dépôt: **06778904.0**

(22) Date de dépôt: **24.07.2006**

(86) Numéro de dépôt international:
**PCT/FR2006/001796**

(87) Numéro de publication internationale:
**WO 2008/003839 (10.01.2008 Gazette 2008/02)**

(54) **UTILISATION D'UNE COMPOSITION COSMETIQUE POUR LUTTER CONTRE LES EFFETS DES ONDES ELECTROMAGNETIQUES SUR LA PEAU**

VERWENDUNG EINER KOSMETISCHEN ZUSAMMENSETZUNG ZUR BEKÄMPFUNG DER WIRKUNGEN VON ELEKTROMAGNETISCHEN STRAHLEN AUF DIE HAUT

USE OF A COSMETIC COMPOSITION FOR COMBATING THE EFFECTS OF ELECTROMAGNETIC WAVES ON THE SKIN

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **06.07.2006 FR 0606144**

(43) Date de publication de la demande:
**01.04.2009 Bulletin 2009/14**

(73) Titulaire: **Laboratoires Clarins
92200 Neuilly sur Seine (FR)**

(72) Inventeur: **COURTIN, Olivier
92100 Boulogne Billancourt (FR)**

(74) Mandataire: **Corizzi, Valérie et al
Cabinet ORES,
36, rue de Saint Pétersbourg
75008 Paris (FR)**

(56) Documents cités:
**WO-A2-2006/004695    FR-A1- 2 843 879
FR-A1- 2 872 043**

- **"Le spectre des ondes électromagnétiques"[Online] 15 juin 2004 (2004-06-15), XP002421047 Extrait de l'Internet: URL:http://www.bbemg.ulg.ac.be/FR/2Notions /spectreem.html> [extrait le 2007-02-19]**
- **"Ondes électromagnétiques"[Online] XP002421048 Extrait de l'Internet: URL:http: //www.ovnis.atfreeweb.com/i_ondes _electromag.htm> [extrait le 2007-02-19]**

**Description**

La présente invention concerne l'utilisation d'une composition cosmétique pour lutter contre les effets des ondes électromagnétiques générées par les téléphones portables sur la peau et les phanères.

La peau d'une surface de 2m$^2$ environ est le plus grand organe de l'organisme. Indispensable à la vie, véritable interface entre le corps et l'environnement, c'est une barrière interactive. Elle possède de grandes capacités d'adaptation ou de réactions face aux agressions. Elle est aussi un site privilégié d'absorption des polluants de l'environnement, avec passage de substances pouvant avoir un effet toxique cutané direct.

La pollution peut revêtir différents aspects : outre la pollution particulaire et gazeuse bien documentée s'ajoute également une nouvelle forme de pollution par les ondes électromagnétiques. La pollution ne se limite pas à l'extérieur, elle concerne également l'intérieur des locaux : l'utilisation de matériaux synthétiques et/ou de produits chimiques à usage domestique, génère de nouveaux polluants. Les principaux agents polluants sont les particules et l'ozone qui génèrent des radicaux libres.

La peau est en contact direct avec les agents polluants. Les dermatologues sont confrontés, dans les zones urbaines, à une recrudescence des peaux irritées et sensibles et à une aggravation de certaines dermatoses.

De nombreuses études ont démontré le potentiel irritant des agents polluants lorsqu'ils sont en contact avec la peau. Ils ont une action sur la modification de la composition en lipides de la peau et acidifient son pH. Ces différentes modifications entrainent une perturbation de la fonction barrière de la peau et une induction de réactions de nature oxydative et inflammatoire, au niveau des couches vivantes de l'épiderme avec expression de protéines de stress, de cytokines pro-inflammatoires et de métalloprotéinases.

Les ondes électromagnétiques sont une forme d'énergie constituée des vibrations des champs électriques et magnétiques. Nous ne les voyons pas, ne les sentons pas, mais elles sont de plus en plus présentes dans notre environnement domestique.

Dans nos habitations, nous sommes exposés aux champs magnétiques générés par les appareils et par les installations électriques domestiques (four micro-ondes, télévision, radio, portable).

A partir d'un seuil plus ou moins élevé, selon les sensibilités individuelles, ces champs électromagnétiques peuvent influer sur notre métabolisme.

Les conséquences des ondes électromagnétiques sur la peau sont moins connues. Pour évaluer leurs effets au niveau cutané, la Demanderesse a mis au point un modèle original permettant d'exposer un épiderme reconstruit in vitro à des ondes électromagnétiques d'une fréquence donnée. Les travaux de la Demanderesse ont permis de mettre en évidence une augmentation de la production des protéines de choc thermiques en réponse au stress, une baisse de la synthèse de β-défensine II, une baisse de la synthèse de filaggrine et de loricrine, et donc principalement une baisse de la différentiation épidermique.

La différenciation terminale de l'épiderme est un processus vectorisé, au cours duquel le kératinocyte -issu de l'assise basale germinative de l'épithélium- subit des remaniements progressifs métaboliques et structuraux qui le transforment finalement en cornéocyte. L'empilement de ces cornéocytes constitue la couche cornée, la partie la plus externe de l'épiderme qui assure -par sa grande résistance- la protection mécanique de l'organisme et constitue une barrière entre l'individu et son environnement en s'opposant aux déperditions hydriques et à la pénétration des molécules exogènes. La couche cornée ainsi formée, résistante et imperméable, permet de limiter la perte insensible en eau et donc de conférer à l'épiderme une bonne hydratation de ses couches supérieures donnant à la peau la souplesse et l'élasticité des peaux qualifiées de 'peaux normales'.

Une baisse de la différentiation épidermique entraine une mauvaise architecture du stratum comeum. Cette architecture perturbée a pour conséquence une cohésion réduite de la barrière cutanée, qui devenant moins imperméable, protège moins efficacement la peau des attaques extérieures. Il s'en suit une baisse de l'hydratation cutanée, une baisse de la souplesse et de l'élasticité de la peau.

Les effets des ondes sur le modèle épidermique se sont traduits par une baisse de la filaggrine et de la loricrine. Le marqueur retenu pour suivre l'effet de la baisse de la différentiation épidermique est la loricrine. En effet, cette protéine est le constituant majeur à 66% de la membrane des cornéocytes (The journal of biological chemistry, vol 270, N°30, p 17702-17711, 1995). Une baisse de la loricrine dans le stratum corneum a pour conséquence une désorganisation importante de son architecture et donc une mauvaise cohésion de la barrière épidermique.

La présente invention concerne donc l'utilisation d'au moins un activateur de la différenciation épidermique pour lutter contre les effets sur la peau des ondes électromagnétiques générées par les téléphones portables, ledit activateur étant un extrait d'origine végétale ou marine choisi parmi un extrait de *Rhodiola rosea,* un extrait de *Thermus thermophillus,* un extrait de *Cedrelopsis grevei,* un extrait de *Salicomia herbacea,* un extrait peptidique de noisette ou le caprylyl butyrate, et ledit activateur de la différenciation épidermique étant appliqué sur la peau sous la forme d'une composition cosmétique.

On entend par activateur de la différentiation épidermique un actif qui permet d'augmenter significativement la différentiation épidermique par rapport au témoin suivant la technique d'immunomarquage de la cytokératine et de la filaggrine plus précisément décrite dans la partie exemple. On analyse l'effet d'un actif sur la synthèse de la cytokératine 10 et de la filaggrine, représentant des marqueurs de différenciation épidermique, dans des cultures de kératinocytes humains en monocouche. Les 2 marqueurs de la différenciation sont révélés par immunocytochimie. L'intensité de la fluorescence permet de localiser la présence ou non des marqueurs.

L'activateur de la différentiation épidermique selon l'invention est d'origine végétale ou marine. Les activateurs de la différentiation épidermique sont choisis parmi un extrait de *Salicornia herbacea,* comme par exemple celui commercialisé par la société Codif sous la dénomination 'huile de Salicorne', un extrait peptidique de noisette, comme par exemple celui commercialisé par la société Solabia sous la dénomination 'Nutéline C', le caprylyl butyrate. De façon préférentielle on choisit un extrait de *Rhodiola rosea,* comme par exemple celui commercialisé par la société Arch Personal Care sous la dénomination 'Rhodiola rosea extract', un extrait de *Thermus thermophillus,* comme par exemple celui commercialisé par la société Sederma sous la dénomination 'Vénucéane®' et un extrait de *Cedrelopsis grevei,* comme par exemple celui commercialisé par la société Bayer Healthcare sous la dénomination 'extrait de Cedrelopsis grevei'.

*Rhodiola rosea* est une plante vivace native des hautes altitudes des régions antarctiques de l'Est Sibérien : elle pousse sur un sol sablonneux, rocheux, froid et sec. Elle a une grande capacité à résister aux stress chimiques, biologiques ou physiques. Cette plante est riche en composés phénoliques qui ont un fort pouvoir anti-oxydant mais aussi en flavonoïdes, salidroside, rosavine, rosine et rosarine.

De façon avantageuse, l'extrait utilisable dans le cadre de la présente invention est issu des racines de la plante et, de préférence, il s'agit d'un extrait hydro-glycolique. Après séchage, les racines de *Rhodiola rosea* sont macérées dans un mélange de solvants eau et pentylène glycol et chauffées à une température de 50°C environ. Une filtration adéquate finalise l'extrait qui se présente sous la forme d'un liquide de couleur ambrée et d'odeur caractéristique. Il présente les caractéristiques analytiques suivantes :

- ph: 4.0 - 6.0
- pourcentage de matière active : 0.6 - 1.2%

De façon avantageuse, l'extrait utilisable dans le cadre de la présente invention peut également être un extrait obtenu par microculture d'un organisme marin : le *Thermus thermophillus.* Ce micro-organisme vit dans les profondeurs sous-marines à 2000m de profondeur, dans la baie de Guayamas dans le Golfe de Californie, à 75°C, à une pression de 200 bars et dans des concentrations importantes en soufre et métaux lourds : des conditions extrêmes de température et un environnement très pro-oxydant.

De façon avantageuse, l'extrait utilisable dans le cadre de la présente invention est obtenu par biotechnologie. Les bactéries *Thermus thermophillus* sont cultivées sur un milieu standard de fermentation, le filtrat ainsi obtenu est concentré par ultrafiltration tangentielle et finalement filtré. L'extrait se présente sous la forme d'un liquide jaune brun limpide à légèrement opalescent, d'odeur caractéristique. Il présente les caractéristiques suivantes :

- ph : 7.0 - 8.0
- densité : 1.010-1.030
- indice de réfraction : 1.335 - 1.355

Enfin, de façon avantageuse, l'extrait utilisable dans le cadre de la présente invention peut également être un extrait de *Cedrelopsis grevei. Cedrelopsis grevei* est un arbre de 5 à 22 m endémique de Madagascar et de la famille des ptaeroxylacées. Il se localise surtout dans les forêts denses et sèches entre 100 et 800 m d'altitude.

De façon avantageuse l'extrait de *Cedrelopsis grevei* utilisé selon l'invention est un extrait d'écorce de l'arbre, et de préférence, il s'agit d'un extrait hydro-glycolique ou hydro-glycériné. Après avoir été séchée, l'écorce broyée est macérée dans un mélange alcool et eau. Après évaporation de l'alcool et réajustement avec du butylène glycol, l'extrait est filtré stérilement. L'extrait de *Cedrelopsis grevei* se présente sous la forme d'un liquide ambré d'odeur caractéristique.

La composition selon l'invention peut contenir un ou plusieurs extraits végétaux ou marins activateurs de la différentiation épidermique. Plus particulièrement, la composition selon l'invention peut contenir un ou plusieurs activateurs choisis parmi un extrait de *Salicornia herbacea,* un extrait peptidique de noisette ou le caprylyl butyrate. Et tout préférentiellement la composition selon l'invention peut contenir un ou plusieurs extraits choisis parmi un extrait de *Rhodiola rosea,* un extrait de *Thermus thermophillus* et un extrait de *Cedrelopsis grevei.*

La composition selon l'invention contient :

- de l'ordre de 0,01 à 10 % en poids, et de préférence 0,01 à 5 % en poids d'extrait de *Rhodiola rosea* et/ou
- de l'ordre de 0,01 à 10 % en poids, de préférence 0,01 à 5 % en poids d'extrait de *Thermus thermophillus* et/ou
- de l'ordre de 0,01 à 10 % en poids, de préférence 0,01 à 5 % en poids d'extrait de *Cedrelopsis grevei.*

La composition cosmétique de la présente invention à application topique peut constituer notamment une composition de protection, de traitement ou de soin cosmétique ou dermatologique pour le visage, pour le cou, pour les mains ou pour le corps, comme, par exemple, crèmes de jour, crèmes de nuit, crèmes ou huiles solaires, laits corporels, une composition capillaire (par exemple lotion capillaire), une composition de maquillage (par exemple fond de teint) ou une composition auto bronzante. La composition cosmétique de la présente invention est, dans une forme de mise en oeuvre préférée, un soin de jour.

La composition cosmétique selon la présente invention peut contenir un ou plusieurs autres composants connus de l'homme du métier, comme des agents de formulation ou additifs d'usage connu et classique dans les compositions cosmétiques. A titre d'exemple et de façon non limitative, de tels agents de formulation et additifs peuvent être des gélifiants hydrophiles ou lipophiles, des adoucissants, des colorants, des agents solubilisants, des agents de texture, des parfums, des charges, des absorbeurs d'odeur, des actifs filmogènes, des conservateurs, des tensio-actifs, des émulsionnants, des huiles, des glycols, des vitamines, des filtres solaires. Grâce à ses connaissances en matière de cosmétiques, l'homme du métier saura quels agents de formulation ajouter à la composition cosmétique selon l'invention et en quelles quantités en fonction des propriétés recherchées.

De plus, la composition cosmétique selon la présente invention peut se présenter sous toute forme connue de l'homme du métier dans le domaine de la cosmétique sans aucune autre restriction galénique particulière que celle pour l'application sur la peau. Ainsi, la composition cosmétique selon l'invention peut avoir la forme d'une solution ou suspension aqueuse, alcoolique ou d'une suspension huileuse ou d'une solution ou d'une dispersion de type lotion ou sérum, d'une émulsion de consistance liquide ou semi-liquide de type lait, obtenues par dispersion d'une phase grasse dans une phase aqueuse (émulsion Huile dans Eau : H/E) ou inversement (Eau dans Huile : E/H), ou d'une émulsion du type crème H/E ou E/H ou d'un gel, d'une lotion, d'un masque. On peut également envisager les formulations cosmétiques selon l'invention sous la forme d'une mousse ou encore sous forme de compositions pour aérosol comprenant également un agent propulseur sous pression.

La présente invention concerne également un procédé de traitement cosmétique pour lutter contre les effets sur la peau des ondes électromagnétiques générées par les téléphones portables, comprenant l'application sur la peau ou les phanères, d'une composition cosmétique comprenant au moins un activateur de la différenciation épidermique, ledit activateur étant un extrait d'origine végétale ou marine choisi parmi un extrait de *Rhodiola rosea,* un extrait de *Thermus thermophillus,* un extrait de *Cedrelopsis grevei,* un extrait de *Salicomia herbacea,* un extrait peptidique de noisette ou le caprylyl butyrate. Ledit activateur est choisi préférentiellement parmi un extrait de *Rhodiola rosea,* un extrait de *Thermus thermophillus* et un extrait de *Cedrelopsis grevei.*

Les exemples ci-après concernent, d'une part, l'évaluation d'une activité pro-différentiation épidermique, et d'autre part l'évaluation de l'effet des ondes électromagnétiques sur la peau, ainsi que l'évaluation de la protection apportée par l'utilisation d'un extrait de *Rhodiola rosea* et de *Thermus thermophillus.* Ils concernent également des compositions objets de la présente invention.

Les exemples font références aux figures suivantes dans lesquelles :

- La figure 1 représente une photo du témoin non exposé, contrôle sans anti-loricrine.
- La figure 2 représente une photo du témoin non exposé, avec marquage de la loricrine.
- La figure 3 représente une photo du témoin exposé aux ondes avec marquage de la loricrine 18 heures après l'exposition.
- La figure 4 représente le témoin 18 heures après l'exposition.
- La figure 5 représente l'épiderme traité par le mélange de l'extrait de *Thermus thermophillus* et de l'extrait de *Rhodiola rosea* et exposé aux ondes, avec marquage de la loricrine 18 heures après l'exposition.

## I. EVALUATION D'UNE ACTIVITE PRO-DIFFERENTIATION EPIDERMIQUE

Le protocole permet d'analyser l'effet d'un actif sur la synthèse de la cytokératine 10 et de la filaggrine, représentant des marqueurs de la différenciation épidermique, dans des cultures de kératinocytes humains en monocouche.

Après traitements dans des milieux nutritifs pendant 4 jours, les 2 marqueurs de la différenciation sont révélés par immunocytochimie.

A. Matériel et méthodes.

1. Cellules utilisées

- Type : kératinocytes épidermiques humains

- Culture : 37°C, 5% $CO_2$
- Milieu : milieu DMEM (Dulbecco Modified Essential Medium)+10%SVF (sérum de veau foetal)

<u>2. Protocole</u>

Les cellules sont rincées et incubées pendant 24 heures dans un milieu DMEM contenant 2mM de L-glutamine et 10% de SVF, milieu nutritif utilisé pour l'étude. Les cellules sont rincées et incubées 96 heures avec l'actif à tester aux différentes concentrations choisies.

Chaque tapis cellulaire est ensuite rincé, fixé au méthanol (-20°C) avant de procéder à la révélation de la filaggrine et de la cytokératine 10 par immunofluorescence.

<u>B. Résultats.</u>

Observée en lumière ultraviolette, la fluorescéine fixée sur les marqueurs fluoresce en vert et permet de les localiser.

<u>II. EVALUATION DE L'EFFET DES ONDES ELECTROMAGNETIQUES DE FREQUENCE 900MHZ SUR DES EPI-DERMES RECONSTRUITS.</u>

<u>A. Matériel et méthodes.</u>

<u>1. Exposition aux ondes électromagnétiques.</u>

Un modèle d'épiderme reconstruit a été exposé pendant 6 heures à des ondes de 900MHz dans une chambre isolée afin de n'avoir aucune autre onde électromagnétique que celle de la fréquence souhaitée. Les ondes de 900MHz choisies pour l'expérimentation correspondent aux ondes les plus fréquentes des téléphones portables.

Après exposition, les épidermes sont incubés à 37°C afin d'évaluer les perturbations engendrées par l'exposition. Un épiderme est incubé pendant 18 heures : il est comparé à un épiderme témoin non exposé.

Pour déterminer les effets des ondes électromagnétiques de fréquence 900MHz sur l'épiderme reconstruit in vitro, la technique de la puce à ADN a été utilisée. Cette technique récente permet de déterminer l'éventuelle activation ou répression de l'expression des gènes. La puce choisie permet de suivre l'expression de 600 gènes majeurs de l'épiderme. Une confirmation par la technique RT-PCR, ou PCR par transcription inverse, a ensuite été réalisée.

<u>2. Echantillons utilisés.</u>

<u>a) Echantillons pour puce à ADN et RT-PCR.</u>

- T1 (6h) et T2 (6h) : témoins non exposés, prélevés après 6h (= témoins 6h).
- EXP1 (6h) et EXP2 (6h) : exposés pendant 6h et prélevés immédiatement après (= exposés 6h).
- T3 (6h+18h) et T4 (6h+18h) : témoins non exposés pendant 6h et prélevés après 18h d'incubation supplémentaire (= témoins 6h+18h).
- EXP3 (6h+18h) et EXP4 (6h+18h) : exposés pendant 6h et prélevés après 18h d'incubation supplémentaire (= exposés 6h+18h).

<u>b) Echantillons pour immunohistochimie</u>

Lots d'épidermes exposés pendant 6h et prélevés après 18h d'incubation.

- lot d'épidermes non exposés (contrôles)
- lot d'épidermes exposés (témoins exposés)
- lot d'épidermes traités par un extrait de *Rhodiola rosea* et un extrait de *Thermus thermophillus* et exposés

<u>3. Effets sur l'expression des gènes - puce à ADN</u>

L'analyse de l'expression des gènes par puce à ADN a été réalisée sur membranes « Custom ATLAS BA600/1 » sélectionnées pour leur importance dans la physiologie cutanée.

La méthodologie utilisée est celle préconisée par Clontech (Palo Alto, USA protocole n° PT3140-1 version n° PR0X591 - http://www.clontech.com/clontech/techinfo/manuals/PDF/PT3140-1.pdf). L'extraction/purification de l'ARN de chaque échantillon a conduit à l'isolement de quantités d'ARN total adéquates.

Les solutions d'ARN total, contenant toutes de l'enzyme RNAse OUT afin d'inhiber les éventuelles RNases, ont été traitées par la DNAse I selon la procédure préconisée par Ambion (ref 1906 - manual version 0503 (htto://www.ambion.com/techlib/prot/bp 1906.pdf), pour éliminer toute trace d'ADN contaminant l'ARN.

La qualité de l'ARN a ensuite été vérifiée sur gel d'agarose pour vérification de la qualité, de la quantité et de l'absence d'ADN).

Les ARNs provenant des échantillons ayant subi le même traitement ont été poolés.

L'étape suivante a été la purification des pools d'ARN messagers (ARNm) par hybridation des extrémités poly (A) des ARNm à des amorces oligo(dT) biotinylées et capture sélective sur billes de streptavidine, selon le protocole AtlasPure (Clontech). Les sondes ADN multiple marquées au $^{33}$P ont été réalisées par transcription inverse des ARNm liés sur billes de poly(dT), à l'aide d'un lot de primaires spécifiques des séquences immobilisées sur les puces, en présence de [$\alpha^{33}$P]-dATP. Cette étape a utilisé les réactifs et le protocole préconisé par Clontech. Les sondes marquées ont été purifiées par chromatographie sur colonnes d'exclusion, la qualité et l'équivalence des sondes marquées a été évaluée par comptage en scintillation liquide.

Deux séries de deux membranes « Custom ATLAS BA600/1 » ont été prétraitées puis les ADNc immobilisés sur chaque membrane ont été hybridés (68C, une nuit) avec les sondes marquées correspondantes; les filtres ont ensuite été lavés extensivement (68C) et placés dans des sacs plastique individuels pour analyse.

- Série n°1 : membrane n°1 - Témoins non exposés, prélevés après 6h = témoins 6h.
  membrane n°2 - Exposés pendant 6h et prélevés immédiatement après = exposés 6h.
- Série n°2 : membrane n°1 - Témoins non exposés pendant 6h et prélevés après 18h d'incubation supplémentaire = témoins 6h+18h
  membrane n°2 - Exposés pendant 6h et prélevés après 18h d'incubation supplémentaire = exposés 6h+18h.

L'analyse a eu lieu par quantification directe de la radioactivité des spots à l'aide d'un appareil PhosphorImager Cyclone (Packard instruments; 3 h, puis 72 h d'acquisition) et du logiciel BD AtlasImage™ 2.7 (BD Biosciences Clontech).

4. Effets sur l'expression de la loricrine RT-PCR.

La confirmation de l'expression du marqueur sélectionné, la loricrine, a été évaluée par RT-Q-PCR.

a) Reverse transcription.

Réalisation de la réaction de transcription inverse des ARNm, en présence de l'amorce oligo(dT) et de l'enzyme Superscript II (Gibco). Puis quantification, par fluorescence, de l'ADNc synthétisé et ajustement des concentrations. Une nouvelle quantification de chaque ADNc, après dilution finale, est réalisée avant la réaction de PCR.

b) PCR quantitative.

Les réactions de PCR ou ACP (amplification en chaîne par polymérase) ont été réalisées par PCR quantitative avec le système « Light Cycler » (Roche Molecular Systems Inc.) et selon les procédures recommandées par le fournisseur.

Ce système d'analyse permet de réaliser des réactions de PCR rapides et performantes, moyennant une mise au point préalable des conditions d'analyse des différents primaires. Il est formé de deux composants principaux :

- *un thermocycleur :* optimisé grâce à l'utilisation de capillaires en verre et à des transferts thermiques extrêmement rapides.
- *un fluorimètre :* permettant de mesurer en continu l'intensité de fluorescence incorporée dans l'ADN (détection à 521 nm).

Le mélange réactionnel (10 $\mu$l final) introduit dans des capillaires pour chaque échantillon est le suivant :

- 2.5 $\mu$l d'ADNc dilué au 1/10$^e$.
- amorces des différents marqueurs utilisés
- Mélange réactionnel (Roche) contenant l'enzyme taq DNA polymérase, le marqueur SYBR Green I (fluorophore qui s'intercale dans l'ADN double brin, au cours de l'étape d'élongation) et du MgCl$_2$.

Les conditions de PCR sont les suivantes :

- Activation : 10 min à 95C
- Réactions de PCR : [10 sec à 95°C, 5 sec à 64°C et 35 sec à 72°C] 40 cycles.
- Fusion : 5 sec à 95°C puis 5 sec à 60°C.

c) Analyse des Q-PCR.

L'incorporation de fluorescence dans l'ADN amplifié est mesurée en continu au cours des cycles de PCR. Ce système permet d'obtenir des courbes de mesure de la fluorescence en fonction des cycles de PCR et d'évaluer ainsi une valeur d'expression relative pour chaque marqueur.

La valeur de RE (relative expression) est exprimée en unités arbitraires selon la formule suivante :

$$(1/2^{nombre\ de\ cycles}) \times 10^6$$

5. Effets sur l'expression de la loricrine-immunohistochimie

Des coupes transversales des épidermes ont été réalisées au microtome (épaisseur 5μm, 2 lames par épiderme, plusieurs coupes par lames) puis maintenues à température ambiante jusqu'à la réalisation des marquages.

Les coupes ont été déparaffinées dans l'acétone glacial pendant 10 minutes à -20°C puis rincées à l'eau distillée. Les marquages immunohistochimiques ont été réalisés à l'aide de l'automate Austostainer (DakoCytomation) et du kit LSAB+ (DakoCytomation K067911). Brièvement, les lames ont été traitées à l'eau oxygénée afin de bloquer les peroxydases endogènes, rincées puis incubées pendant 30 minutes à température ambiante dans la solution d'anticorps primaire, anti-lorierine. Après lavages, les coupes ont été incubées pendant 15 minutes à température ambiante avec la solution d'anticorps secondaires couplés à la biotine, à nouveau rincées puis incubées pendant 15 minutes avec la solution de peroxydase couplée à la streptavidine. La révélation enzymatique a été réalisée à l'aide du mélange substrat/chromogène du kit LSAB+. Les lames ont ensuite été lavées, contre-colorées avec une solution d'hématoxyline (DakoCytomation S3309), rincées à l'eau distillée puis montées en milieu aqueux Glycergel (DakoCytomation C0563).

Les coupes ont été observées à l'aide d'un microscope inversé NIKON Diaphot 300 (objectif x40). Les saisies d'images ont été réalisées à l'aide d'une caméra COHU pilotée par un logiciel Lucia 7.0.

B. Résultats.

1. Expression du gène de la loricrine - puce à ADN

Les résultats sont exprimés en unités relatives d'expression (RE). Ces niveaux sont corrigés du bruit de fond moyen présent sur chaque membrane et des différences d'intensité de marquage des différentes sondes utilisées (cette correction est réalisée sur la base des différences d'intensité de marquage des gènes de référence).

Dans cette expérience, l'exposition des épidermes aux ondes diminuait de 38% le niveau d'expression de la loricrine 2 heures après l'exposition et de 28 % 18 heures après l'exposition. Ces variations, de faible intensité, nécessitaient d'être vérifiées par RT-PCR quantitative.

2. Expression du gène de la loricrine -RT-PCR

L'analyse quantitative du taux d'ARNm correspondant à la loricrine a été réalisée par RT-PCR quantitative. L'exposition des épidermes aux ondes diminuait de 20 % le niveau d'expression de la loricrine 2 heures après l'exposition et l'augmentait de 20 % 18 heures après l'exposition.

3. Expression du gène de la loricrine-Immunohistochimie

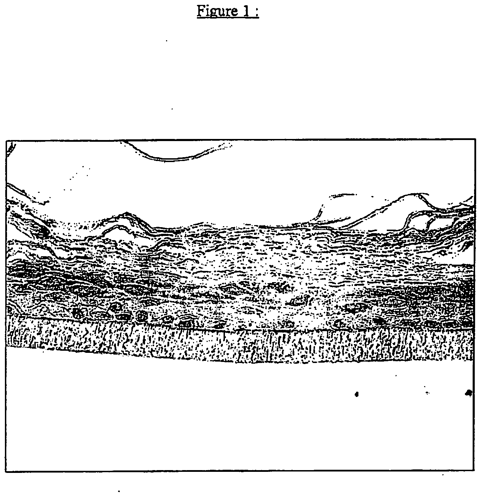

a) Contrôle anticorps secondaire seul (figure 1)

Comme attendu, aucun signal n'a été observé au sein de l'épiderme lorsque le marquage a été réalisé en présence de l'anticorps secondaire seul (sans anticorps primaire anti-loricrine).

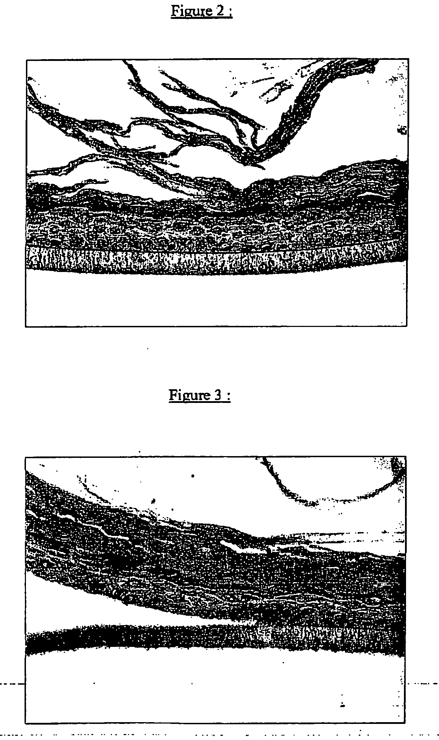

b) Effet de l'exposition aux ondes (figures 2 et 3)

Le marquage anti-loricrine était principalement localisé au niveau de la couche granuleuse. Le marquage était moins intense et discontinu dans les épidermes exposé aux ondes téléphoniques.

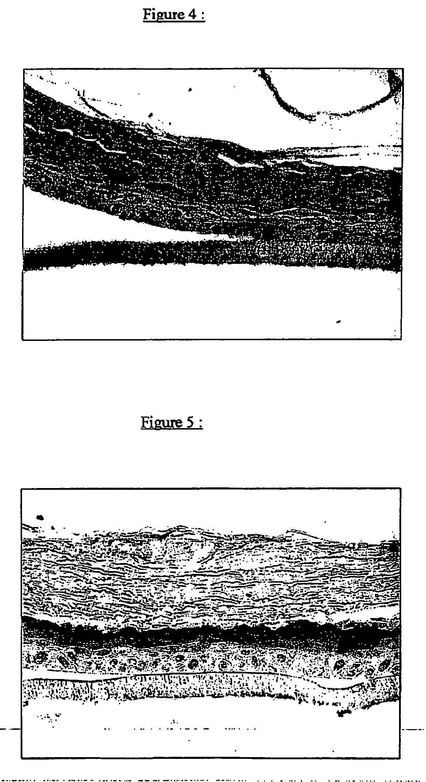

c) Effet de l'extrait de *Rhodiola rosea* et de l'extrait de *Thermus thermophillus* (figures 4 et 5)

Dans ces conditions expérimentales, le traitement des épidermes exposés aux ondes par le produit « Actif A+B » a augmenté l'intensité du marquage par rapport au témoin exposé. Le marquage pourrait être supérieur à celui du témoin non exposé.

C. Conclusion.

La technique de la puce à ADN est puissante pour détecter les effets de traitements sur le niveau d'expression de nombreux gènes dans un seul essai, elle nous a donc permis de faire un premier criblage. Elle présente par contre l'inconvénient de ne pas être quantitative et de n'être fiable que pour des amplitudes de variation d'expression importante. A l'inverse la technique RT-PCR quantitative présente l'avantage de mesurer avec précision le taux d'ARN messager d'un gène donné. Dans les essais réalisés, l'analyse par la puce à ADN indiquait une diminution d'expression de la loricrine après l'exposition des épidermes aux ondes, alors que la RT-PCR a montré une diminution de 20 % du niveau d'expression de la loricrine 2 heures après l'exposition et une augmentation de 20 % 18 heures après l'exposition. L'analyse par immunomarquage de coupes des épidermes a confirmé une diminution de la loricrine 18 heures après l'exposition. Cette apparente contradiction pourrait s'expliquer par le fait que la diminution d'ARNm à 2 heures ne se traduit au niveau protéique que plus tardivement.

Les extraits de *Rhodiola rosea* et de *Thermus thermophillus* ont montré une nette protection contre cet effet inhibiteur des ondes sur l'expression de la loricrine, et donc de la différentiation épidermique.

III. EXEMPLES

A. GEL CREME ANTI-ONDES

|                                   | %         |
|-----------------------------------|-----------|
| EAU DEMINERALISEE                 | q.s.p 100 |
| PEMULEN TR1                       | 0,5       |
| GLYCERINE                         | 5,0       |
| SEPIGEL 305                       | 1,0       |
| EXTRAIT DE RHODIOLA ROSEA         | 2,0       |
| EXTRAIT DE THERMUS THERMOPHILLUS  | 2,0       |
| ISONONYL ISONONANOATE             | 7,0       |
| C12 - C15 ALKYL BENZOATE          | 3,0       |
| HUILE DE SILICONE                 | 2,0       |
| HYDROXYDE DE SODIUM               | 0,2       |
| PARFUM                            | 0,3       |
| COLORANTS A 1 %                   | 0,12      |
| CONSERVATEURS                     | 1,0       |

B.CREME ANTI-ONDES

|                       | %         |
|-----------------------|-----------|
| EAU DEMINERALISEE     | q.s.p 100 |
| CETEARYL GLUCOSIDE    | 5,0       |
| TRIGLYCERIDES C8-C10  | 10        |

(suite)

|  | % |
|---|---|
| HUILE DE SILICONE | 2,0 |
| SILICONE VOLATILE | 5,0 |
| CARBOMERE | 0,2 |
| GLYCERINE | 5,0 |
| EXTRAIT DE RHODIOLA ROSEA | 2,0 |
| EXTRAIT DE THERMUS THERMOPHILLUS | 2,0 |
| PARFUM | 0,3 |
| CONSERVATEURS | 1,0 |

C.LOTION POUR LA PEAU ANTI-ONDES

|  | % |
|---|---|
| EAU DEMINERALISEE | q.s.p 100 |
| EDTA DISODIQUE | 0,2 |
| CHLORURE DE SODIUM | 1,0 |
| EXTRAIT DE RHODIOLA ROSEA | 2,0 |
| EXTRAIT DE THERMUS THERMOPHILLUS | 2,0 |
| EXTRAIT DE CEDRELOPSIS GREVEI | 2,0 |
| EAU DE ROMARIN | 4,0 |
| GLYCOL | 1,0 |
| CONSERVATEURS | 1,0 |

D.LOTION CAPILLAIRE ANTI-ONDES

|  | % |
|---|---|
| DIMETHICONE | 8,00 |
| SOLUTION DE COLORANT | 0,60 |
| HUILES ESSENTIELLES | 0,40 |
| ALCOOL 96.2° NON DENATURE | 25,00 |
| EXTRAIT DE CEDRELOPSIS GREVEI | 2,00 |
| EXTRAIT DE RHODIOLA ROSEA | 2,00 |
| EXTRAIT DE THERMUS THERMOPHILLUS | 2,00 |
| PARFUM | 1,00 |
| EAU DEMINERALISEE | Q.s.P 100 |

E.FOND DE TEINT ANTI-ONDES

|  | % |
|---|---|
| EXTRAIT DE CEDRELOPSIS GREVEI | 2,00 |
| EXTRAIT DE RHODIOLA ROSEA | 2,00 |
| EXTRAIT DE THERMUS THERMOPHILLUS | 2,00 |
| EMULSIONNANT SILICONE | 5,00 |
| CO-EMULSIONNANT | 0,50 |
| ACIDE SORBIQUE | 0,05 |

(suite)

|  | % |
|---|---|
| OCTYL METHOXYCINNAMATE | 5,00 |
| CIRE DE SILICONE | 3,00 |
| SILICONE VOLATIL | 10,50 |
| GEL DE BENTONE | 2,00 |
| OXYDES DE FER | 13,40 |
| ELASTOMERE DE SILICONE | 2,00 |
| SILICONE POUDRE | 0,90 |
| PARFUM | 0,50 |
| CHLORURE DE SODIUM PUR | 2,00 |
| EDTA | 0,10 |
| CONSERVATEUR | 1,00 |
| SERICITE | 0,20 |
| MICA / TITANE | 0,20 |
| PROPYLENE GLYCOL | 4,10 |
| EAU DEMINERALISEE | Q.s.P 100 |

**Revendications**

1. Utilisation d'au moins un activateur de la différenciation épidermique pour lutter contre les effets sur la peau des ondes électromagnétiques générées par les téléphones portables, ledit activateur étant un extrait d'origine végétale ou marine choisi parmi un extrait de *Rhodiola rosea,* un extrait de *Thermus thermophillus,* un extrait de *Cedrelopsis grevei,* un extrait de *Salicornia herbacea,* un extrait peptidique de noisette ou le caprylyl butyrate, et ledit activateur de la différenciation épidermique étant appliqué sur la peau sous la forme d'une composition cosmétique.

2. Utilisation selon la revendication 1, **caractérisée en ce que** ledit activateur est choisi parmi un extrait de *Rhodiola rosea,* un extrait de *Thermus thermeophillus* et un extrait de *Cedrelopsis grevei.*

3. Utilisation selon la revendication 1 ou 2, **caractérisée en ce que** l'extrait de *Rhodiola rosea* est un extrait issu des racines de la plante.

4. Utilisation selon la revendication 3, **caractérisée en ce que** l'extrait de racines de *Rhodiola rosea* est un extrait hydro-glycolique.

5. Utilisation selon la revendication 1 ou 2, **caractérisée en ce que** l'extrait de *Thermus thermophillus* est un extrait de bactéries *Thermus thermophillus* obtenu par biotechnologie.

6. Utilisation selon la revendication 1 ou 2, **caractérisée en ce que** l'extrait de *Cedrelopsis grevei* est un extrait issu de l'écorce de l'arbre.

7. Utilisation selon la revendication 6, **caractérisée en ce que** l'extrait d'écorce de *Cedrelopsis grevei* est un extrait hydro-glycolique ou hydro-glycériné.

8. Utilisation selon l'une quelconque des revendications 1 à 7, **caractérisée en ce que** la composition contient:

   - de l'ordre de 0,01 à 10 % en poids, et de préférence 0,01 à 5 % en poids d'extrait de *Rhodiola rosea* et/ou
   - de l'ordre de 0,01 à 10 % en poids, de préférence 0,01 à 5 % en poids d'extrait de *Thermus thermophillus* et/ou
   - de l'ordre de 0,01 à 10 % en poids, de préférence 0,01 à 5 % en poids d'extrait de *Cedrelopsis grevei.*

9. Utilisation selon l'une quelconque des revendications précédentes pour lutter contre les effets des ondes de fréquence 900 MHz.

10. Procédé de traitement cosmétique pour lutter contre les effets sur la peau des ondes électromagnétiques générées par les téléphones portables, comprenant l'application sur la peau ou les phanères, d'une composition cosmétique comprenant au moins un activateur de la différenciation épidermique, ledit activateur étant un extrait d'origine végétale ou marine choisi parmi un extrait de *Rhodiola Rosea,* un extrait de *Thermus thermophillus,* un extrait de *Cedrelopsis grevei,* un extrait de *Salicornia herbacea,* un extrait peptidique de noisette ou le caprylyl butyrate.

**11.** Procède selon la revendication 10, **caractérisé en ce que** ledit activateur est choisi parmi un extrait de *Rhodiola rosea,* un extrait de *Thermus thermophillus* et un extrait de *Cedrelopsis grevei.*

**Claims**

**1.** Use of at least one epidermal differentiation activator for combating the effects on the skin of the electromagnetic waves generated by mobile telephones, said activator being an extract of vegetable or marine origin selected from an extract of *Rhodiola rosea,* an extract of *Thermus thermophillus,* an extract of *Cedrelopsis grevei,* an extract of *Salicornia herbacea,* a hazelnut peptide extract and caprylyl butyrate, and said epidermal differentiation activator being applied to the skin in the form of a cosmetic composition.

**2.** Use according to Claim 1, **characterized in that** said activator is selected from an extract of *Rhodiola rosea,* an extract of *Thermus thermophillus* and an extract of *Cedrelopsis grevei.*

**3.** Use according to Claim 1 or 2, **characterized in that** the extract of *Rhodiola rosea* is an extract derived from the roots of the plant.

**4.** Use according to Claim 3, **characterized in that** the root extract of *Rhodiola rosea* is a hydroglycolic extract.

**5.** Use according to Claim 1 or 2, **characterized in that** the extract of *Thermus thermophillus* is an extract of *Thermus thermophillus* bacteria obtained by biotechnology.

**6.** Use according to Claim 1 or 2, **characterized in that** the extract of *Cedrelopsis grevei* is an extract derived from the bark of the tree.

**7.** Use according to Claim 6, **characterized in that** the bark extract of *Cedrelopsis grevei* is a hydroglycolic or hydroglycerolic extract.

**8.** Use according to any one of Claims 1 to 7, **characterized in that** the composition contains:

- in the order of 0.01 to 10 wt.%, preferably 0.01 to 5 wt.%, of extract of *Rhodiola rosea,* and/or
- in the order of 0.01 to 10 wt.%, preferably 0.01 to 5 wt.%, of extract of *Thermus thermophillus,* and/or
- in the order of 0.01 to 10 wt.%, preferably 0.01 to 5 wt.%, of extract of *Cedrelopsis grevei.*

**9.** Use according to any one of the preceding claims for combating the effects of waves with a frequency of 900 MHz.

**10.** Method of cosmetic treatment for combating the effects on the skin of the electromagnetic waves generated by mobile telephones, comprising the application, to the skin or superficial body growths, of a cosmetic composition comprising at least one epidermal differentiation activator, said activator being an extract of vegetable or marine origin selected from an extract of *Rhodiola rosea,* an extract of *Thermus thermophillus,* an extract of *Cedrelopsis grevei*, an extract of *Salicornia herbacea*, a hazelnut peptide extract and caprylyl butyrate.

**11.** Method according to Claim 10, **characterized in that** said activator is selected from an extract of *Rhodiola rosea,* an extract of *Thermus thermophillus* and an extract of *Cedrelopsis grevei.*

**Patentansprüche**

**1.** Verwendung wenigstens eines Aktivators der epidermalen Differenzierung, um die Wirkungen der elektromagnetischen Wellen, die durch Mobiltelefone erzeugt werden, auf die Haut zu bekämpfen, wobei der Aktivator ein Extrakt pflanzlicher oder mariner Herkunft ist, ausgewählt aus einem Extrakt von *Rhodiola rosea,* einem Extrakt von *Thermus thermophillus,* einem Extrakt von *Cedrelopsis grevei,* einem Extrakt von *Salicornia herbacea,* einem Nusspeptidextrakt oder Caprylylbutyrat, und wobei der Aktivator der epidermalen Differenzierung in Form einer kosmetischen Zusammensetzung auf die Haut aufgetragen wird.

2. Verwendung gemäß Anspruch 1, **dadurch gekennzeichnet, dass** der Aktivator aus einem Extrakt von *Rhodiola rosea*, einem Extrakt von *Thermus thermophillus* und einem Extrakt von *Cedrelopsis grevei* ausgewählt ist.

3. Verwendung gemäß Anspruch 1 oder 2, **dadurch ge - kennzeichnet**, dass der Extrakt von *Rhodiola rosea* ein Extrakt ist, der aus den Wurzeln der Pflanze stammt.

4. Verwendung gemäß Anspruch 3, **dadurch gekennzeichnet, dass** der Extrakt aus Wurzeln von *Rhodiola rosea* ein wässrig-glykolischer Extrakt ist.

5. Verwendung gemäß Anspruch 1 oder 2, **dadurch gekennzeichnet, dass** der Extrakt von *Thermus thermophillus* ein *Thermus thermophillus*-Bakterienextrakt ist, der durch Biotechnologie erhalten wird.

6. Verwendung gemäß Anspruch 1 oder 2, **dadurch gekennzeichnet, dass** der Extrakt von *Cedrelopsis grevei* ein Extrakt ist, der aus der Rinde des Baumes stammt.

7. Verwendung gemäß Anspruch 6, **dadurch gekennzeichnet, dass** der *Cedrelopsis grevei*-Rindenextrakt ein wässrig-glykolischer oder wässrig-glyzerinischer Extrakt ist.

8. Verwendung gemäß einem der Ansprüche 1 bis 7, **dadurch gekennzeichnet, dass** die Zusammensetzung enthält:

   - Extrakt von *Rhodiola rosea* in der Größenordnung von 0,01 bis 10 Ges.-% und vorzugsweise von 0,01 bis 5 Gew.-% und/oder
   - Extrakt von *Thermus thermophillus* in der Größenordnung von 0,01 bis 10 Gew.-%, vorzugsweise 0,01 bis 5 Gew.-%, und/oder
   - Extrakt von *Cedrelopsis grevei* in der Größenordnung von 0,01 bis 10 Gew.-%, vorzugsweise 0,01 bis 5 Gew.-%.

9. Verwendung gemäß einem der vorangehenden Ansprüche, um die Wirkungen von Wellen mit einer Frequenz von 900 MHz zu bekämpfen.

10. Kosmetisches Behandlungsverfahren, um die Wirkungen elektromagnetischer Wellen, die durch Mobiltelefone erzeugt werden, auf die Haut zu bekämpfen, umfassend Auftragung einer kosmetischen Zusammensetzung auf die Haut oder die Hautanhanggebilde, die wenigstens einen Aktivator der epidermalen Differenzierung umfasst, wobei der Aktivator ein Extrakt pflanzlicher oder mariner Herkunft ist, ausgewählt aus einem Extrakt von *Rhodiola rosea,* einem Extrakt von *Thermus thermophillus,* einem Extrakt von *Cedrelopsis grevei*, einem Extrakt von *Salicornia herbacea,* einem Nusspeptidextrakt oder Caprylylbutyrat.

11. Verfahren gemäß Anspruch 10, **dadurch gekennzeichnet, dass** der Aktivator aus einem Extrakt von *Rhodiola rosea,* einem Extrakt von *Thermus thermophillus* und einem Extrakt von *Cedrelopsis grevei* ausgewählt wird.

Figure 1 :

Figure 2 :

Figure 3 :

Figure 4 :

Figure 5 :

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- *The journal of biological chemistry,* 1995, vol. 270 (30), 17702-17711